# EUROPEAN PATENT APPLICATION

(11) **EP 4 358 092 A1**
(43) Date of publication of application: **24.04.2024**
(21) Application number: 22202851.6
(22) Date of filing: 20.10.2022
(51) Int. Cl.: G16H 40/63, G16H 50/30

(54) **NOTIFICATION HANDLING DEPENDENT UPON USER STATE**

(71) Applicant: Koninklijke Philips N.V., 5656 AG Eindhoven (NL)
(72) Inventor: KUHLMANN, Daan, Eindhoven (NL); GELISSEN, Jozef Hubertus, Eindhoven (NL)
(74) Representative: Philips Intellectual Property & Standards

(57) **Abstract**

Some embodiments are directed to a communication device (200) that have a sensor system (210) configured for obtaining sensor data (211) from a user of the communication device. One or more indicators for sleepiness of the user may be determined from the sensor data. The indicators may then be used to select a notification item (241) that is displayed to the user.

## Description

### FIELD OF THE INVENTION

The presently disclosed subject matter relates to a communication device, a method for handling notification items on a communication device, and a computer readable medium.

### BACKGROUND OF THE INVENTION

Many people check their phone first thing in the morning when they are just getting awake, although psychiatrists agree that picking up the phone first thing in the morning can lead to the brain overloading.

Smartphone usage has skyrocketed during the last years and everything, from agenda management to watching series, can be done on a smartphone. It is getting harder to imagine a life without a smartphone and the device is used throughout the day. This usage causes physical, but also psychological problems. One of these problems is stress, because having the option to have all knowledge so close leads to information overload or fear of missing out (FOMO). Especially in the morning, when just getting out of bed, the information influx through the smartphone can lead to an unintended stress spike. For instance, an important agenda item pops up, which needs solving immediately.

Notifications when a user is not in a fit state to handle them may impact the effectiveness of his/her interaction with the device.

Current smartphones provide only limited options to handle notifications. For example, on a known smartphone notifications show when the user swipes down from the top of the screen. Some notifications can also show on the lock screen and home screen. After a notification was displayed, it can be cleared by swiping the notification on the screen left or right. A button may be provided on the bottom of the notifications, marked 'Clear all'; tapping this button clears all notifications.

### SUMMARY OF THE INVENTION

It is desirable to have a smartphone system with an improved notification handling. For example, a smartphone may check a user's readiness and for sleepiness in the morning and derive from this what notification items, e.g., system maintenance, mails, agenda items or notification to show and/or the order in which to show them, so the brain will not overload from the amount of information given. A particularly interesting embodiment uses electro-physiological sensing, with in particular dry electrode sensing. For example, recent dry electrode sensors may be used on or with a smartphone. It will be beneficial for the user to better present notification information.

In an embodiment, one or more indicators for sleepiness of the user are determined from sensor data. An indicator for sleepiness may be a value, e.g., a value selected from a scale. The sensor data may be obtained from a sensor system that may comprise or connect to one or more sensors. The one or more sensors may comprise a camera, in particular a user facing camera, and/or a dry electrode. More generally, a user state may be derived for the user from the sensor data. For example, a user state may comprise one or more indicators for mood. The user state indicating the expected effectiveness of providing notification items to the user at the moment.

A notification item may be selected for displaying to the user from a larger set of notification items. The larger set of notification items may comprise multiple notification items. The larger set of notification items may comprise notification items from multiple sources, e.g., internal and external sources. Selecting a notification item takes into account the sleepiness indicators and/or for other indicators.

In an embodiment, notification items are presented to a notification handler according to an embodiment as a queue, e.g., an intermittingly arriving notification items. Especially in the morning the backlog in the queue may lead to a large number of notification for display. The notification handler can select to suppress notification items according to a user state. The notification handler can also alter the order of a notification queue into a new queue with re-ordered notification items.

Notification handling according to an embodiment may be implemented in a communication device, e.g., a mobile communication device, e.g., a smartphone, table, laptop, and the like.

In addition to sleepiness, many physiological parameters may be determined from sensor data for the user. Such physiological parameters may also or instead be taken into account when selecting a notification item.

Machine learning is applicable in embodiments. For example, selecting a notification item given a current user state may use a machine learnable model. The model be trained on previous examples of a notification item, a user state prior to display of the notification item, and a user state after display of the notification item.

For example, determining a user state from sensor data may be done with a machine learnable model. For example, the machine learnable model may be trained on sensor data and known user states.

A notification item handling method may be implemented on an electronic device. A communication device is an electronic device. Examples of suitable electronic device include a mobile electronic device, e.g., a mobile phone, a computer, etc.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter is a method of making the computer program available for downloading.

### BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Fig. 1 schematically shows an example of an embodiment of a communication device,
Fig. 2 schematically shows an example of an embodiment of a communication device,
Fig. 3 schematically shows an example of an embodiment of a set of notification items,
Fig. 4 schematically shows an example of an embodiment of a method for handling notification items,
Fig. 5a schematically shows a computer readable medium having a writable part comprising a computer program according to an embodiment,
Fig. 5b schematically shows a representation of a processor system according to an embodiment.

### Reference signs list

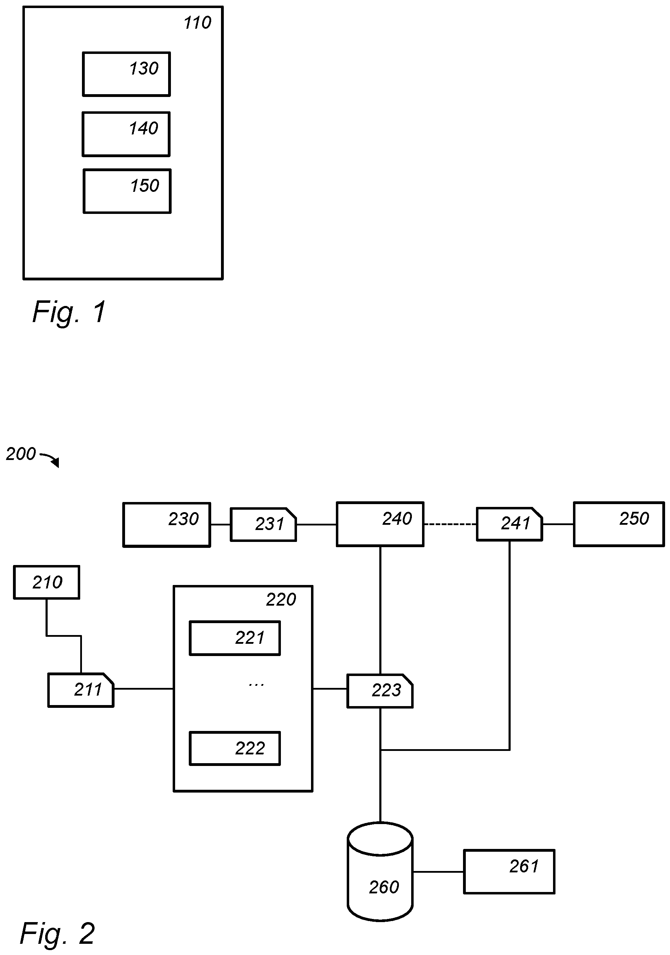

The following list of reference signs refers to Figs. 1-3, 5a, 5b, and is provided for facilitating the interpretation of the drawings and shall not be construed as limiting the claims.
- 110: a communication device
- 130: a processor system
- 140: storage
- 150: communication interface
- 200: a communication device
- 210: a sensor system
- 211: sensor data
- 220: an indicator unit
- 221: a sleepiness unit
- 222: a mood unit
- 223: a user state
- 230: a notification items obtainer
- 231: a set of notification items
- 240: a selector
- 241: a notification item
- 250: a display
- 260: a user response storage
- 261: a machine learning unit
- 310: a set of notification items
- 311-314: a notification item
- 320: a set of notification features
- 321-334: notification features
- 340: a set of weights
- 341-344: a weight
- 350a-350c: a set of selected notification items
- 361: an inserted notification item

- 1000: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF THE EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

**Fig. 1** schematically shows an example of an embodiment of a communication device 110. Communication device 110 is configured to collect notification items and displaying, e.g., all, or at least one, to a user. The selection of which notification items to display, or to display now, and/or the order in which to display them depends on a state of the user, e.g., a physiological state, such as the sleepiness or mood of the user. The user state may be determined from sensor data. For example, the communication device 110 is configured to improve the interaction between the user and the device. Notification items that are displayed when the user is not in a fit state to receive them will receive in fewer or worse interactions with the communication device 110.

For example, a notification item concerning say, a full storage of the communication device 110, which the user ought to act upon, may nevertheless be ignored if the notification item is displayed when the user is too sleepy or is not in the mood for system maintenance.

Communication device 110 may comprise a processor system 130, a storage 140, and a communication interface 150. Storage 140 may be, e.g., electronic storage, magnetic storage, etc. The storage may comprise local storage, e.g., a local hard drive or electronic memory. Storage 140 may comprise non-local storage, e.g., cloud storage. In the latter case, storage 140 may comprise a storage interface to the non-local storage. Storage may comprise multiple discrete sub-storages together making up storage 140. Storage may comprise a volatile writable part, say a RAM, a non-volatile writable part, e.g., Flash, a non-volatile non-writable part, e.g., ROM.

Storage 140 may be non-transitory storage. For example, storage 140 may store data in the presence of power such as a volatile memory device, e.g., a Random Access Memory (RAM). For example, storage 140 may store data in the presence of power as well as outside the presence of power such as a non-volatile memory device, e.g., Flash memory.

The device 110 may communicate internally, with other systems, with other devices, external storage, input devices, output devices, and/or one or more sensors over a computer network. The computer network may be an internet, an intranet, a LAN, a WLAN, etc. The computer network may be the Internet. The device 110 comprise a connection interface which is arranged to communicate within communication device 110 or outside of communication device 110 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 150 may be used to send or receive digital data, e.g., sensor data received from a sensor, e.g., in a sensor system, notification data, electronic communication, technical data concerning device 110 or an associated online account, e.g., a cloud account.

The execution of device 110 may be implemented in a processor system. The device 110 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. Device 110 may comprise multiple processors. A processor circuit may be implemented in a distributed fashion, e.g., as multiple sub-processor circuits. For example, device 110 may use cloud computing.

Typically, the communication device 110 comprises a microprocessor which executes appropriate software stored at the device; for example, that software may have been downloaded and/or stored in a corresponding memory, e.g., a volatile memory such as RAM or a non-volatile memory such as Flash.

Instead of using software to implement a function, device 110 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, communication device 110 may comprise circuits, e.g., for cryptographic processing, and/or arithmetic processing.

In hybrid embodiments, functional units are implemented partially in hardware, e.g., as coprocessors, e.g., neural network coprocessors, and partially in software stored and executed on the device.

**Fig. 2** schematically shows an example of an embodiment of a communication device 200. Communication device 200 is configured to select from a set of notification items 231 a notification item 241 for displaying. The selecting is based on detected indicators for the user, e.g., indicators for sleepiness and/or mood. By taking such user indicators into account, notification items can be displayed in a manner better suited to the current circumstances of the user.

Communication device 200 may be adapted to various kinds of notification items, e.g., items arranged for notification to a user. For example, notification items may include technical communication regarding the functioning of the device. For example, communication regarding the operation of communication device 200 itself or of another device. It is expected that a user of communication device 200 would act on such a notification item, e.g., engage in a technical interaction, e.g., with communication device 200 or with some other device.

The notification items may include various types of notification items. For example, notification items may include one or more of agenda notification, notification items sent by an app installed on the communication device, e-mail messages, communication messages, and so on.

Communication device 200 may comprise a sensor system **210** configured for obtaining sensor data 211 from a user of the communication device. Sensor data 211 may be a snapshot of a user state taken from sensor system 210 whenever communication device 200 needs sensor data for an embodiment. On the other hand, sensor data 211 may be a stream of data obtained from sensor system 210. The stream may be used to continuously estimate a user state. The stream could be obtained only whenever an embodiment is active, or throughout activity of communication device 200.

The sensor system 210 may comprise one or more sensors, e.g., one or more sensor comprised in communication device 200. For example, sensor system 210 may comprise a sensor interface configured to interface with one or more external sensors. The external sensors may include, e.g., a wearable sensor. For example, a user of communication device 200 may wear a sensor included in a device, external to communication device 200. For example, the sensor may be included in a smartwatch.

Regardless of the location of the sensor, e.g., internal or external to communication device 200, the one or more sensors produce sensor data 211 that is indicative of a state of the user. The user state may be a physiological user state, e.g., relating to the organic processes of the user or any of its parts or of a particular bodily process. The user state preferably relates to the normal functions of the living organism, e.g., its functions and activities and of the physical phenomena involved. Many types of sensors are suitable to provide information about the physiological state of the user of communication device 200. A particularly important physiological process in living organism is sleep, and the associated function of sleepiness. Sleepiness may be useful in its function to move a person toward sleeping, but is not conducive to handling a list of notification items.

For example, in an embodiment, communication device 200 and/or sensor system 210 comprises a camera arranged to record an image or video of a face of the user. A camera be used to identify in the recording one or more indicators of sleepiness, mood, heart rate, wakefulness, and so on.

For example, in an embodiment, communication device 200 and/or sensor system 210 comprises a sensor for galvanic skin response sensor (GSR), heartrate, ECG, etc. For example, the sensor may be a dry electrode sensor.

The sensor may more generally measure epidermal skin activity (EDA), in particular electrodermal activity. The galvanic skin response (GSR) is an example of electrodermal activity. For example, changes in sweat gland activity, which may be reflective of the intensity of a user's emotional state or emotional arousal.

GSR and EDA measure the basic phenomenon that skin momentarily becomes a better conductor of electricity on external stimuli. This increased conductivity is due to an increase of activity of sweat glands. Interestingly, there is a correlation between sympathetic nerve activity, e.g., fight-flight response, and skin conductance responses. GSR may be measured by passing a small current through a pair of electrodes placed on the surface of the skin. For example, in an embodiment, communication device 200 may determine that a user picks up communication device 200 and that the GSR changes.

For example, in an embodiment, communication device 200 and/or sensor system 210 comprises a sleep sensor and/or activity tracker configured to determine sleep quality in a night preceding the displaying of the notification item.

For example, in an embodiment, communication device 200 and/or sensor system 210 comprises a photoplethysmography sensor.

Sensor data may also be obtained from usage of communication device 200. For example, communication device 200 may be configured to measure reaction time. For example, a software application on communication device 200 may be configured to measure reaction time. For example, a software application may be configured as a sensor. For example, a sensor may be a hardware element distinct from memory and/or processor of communication device 200.

For example, heart rate may be measured by two electrodes positioned on two outer edges of the smartphone. Whenever the user is holding the smartphone with two hands, communication device 200 may be configured to register heart rate of the user. In an embodiment, and ECG is obtained from the two electrodes. Additionally, heart rate may be measured in a plurality of ways via an external, connected device such as a photoplethysmography (PPG) sensor, e.g., in installed in a band worn around the wrist. A sensor may be installed in the external device, and communication device 200 and/or sensor system may be configured to receive sensor data from the external sensor.

Communication device 200 may comprise **an indicator unit 220** configured to determine from the sensor data one or more indicators for user. A user state 223 may be generated by indicator the unit 220 and may comprise one or more indicators.

For example, user state 223 and/or an indicator may indicate a physiological state of the user. Physiological state may refer to the condition or state of the body or bodily functions. In particular a current condition or state, e.g., the short-term changes therein as opposed to long term changes such as the general health status of the body are important, e.g., the hour to hour or minute to minute changes in a person's physiology. For example, physiological state may include physical condition and/or physiological condition. User state 223 may comprise a wakefulness, e.g., an indication of how conscious and/or aware of the world the user currently is. The user state may comprise a sleepiness, e.g., an indication of how much consciousness of the world is suspended.

In an embodiment, an indicator for user state, e.g., an indicator of sleepiness, and/or an indicator of mood is derived from sensor data obtained from a sensor applied to a user of communication device 200. For example, indicator unit 220 may comprise one or more units configured to derive indicators of particular aspects of a user state. For example, indicator unit 220 may comprise a sleepiness unit 221, and/or a mood unit 222.

An indicator unit such as a sleepiness unit 221, and/or a mood unit 222 may comprise a machine learnable model that was previously trained and which is applied to sensor data 211. For example, the machine learnable model may be trained on data specific to the user of communication device 200 and/or on data collected from other users. For example, in an embodiment, camera images and/or clips may be collected from the user and/or from other users, together with a label that indicates the presence and/or absence of sleepiness. The machine learnable model may be trained to predict the label from the collected data. Once the machine learnable model is trained it may be used by indicator unit 220, e.g., in this case, by sleepiness unit 221, to label new sensor data with an indicator of sleepiness. A mood predicator may be made in a similar way.

In an embodiment, communication device 200 and/or indicator system 220 may be configured to determine from the sensor data one or more physiological parameters for the user, the physiological parameters being indicators for sleepiness of the user. The physiological parameters may be physiological parameters, such as blood pressure, body temperature, breathing rate, heart rate, blood oxygen saturation, and various electrophysiological signals. Physiological parameters are useful as reference values in human health monitoring. From physiological parameters various indicators such as sleepiness and/or mood may be derived.

For example, a **sleepiness unit 221,** which may be comprised in indicator unit 220, may be configured to determine from the sensor data one or more indicators for sleepiness of the user. Sleepiness refers to the inability to stay awake even in situations in which wakefulness is required, such as at work or behind the wheel of a car. Fatigue is a state of overwhelming sustained exhaustion and decreased capacity for physical and mental work that is not relieved by rest. Fatigue may also or instead be determined from sensors data, e.g., by a fatigue unit (not shown).

Sleepiness can be detected with a front facing camera of communication device 200, e.g., a smartphone, tablet, or a webcam. For example, sleepiness unit 221 may be configured to identify in camera sensor data sleepiness indicators, including, e.g., eyebags, eye blinking and openness, nodding and yawning. Apart from the visual indicators, other indicators of sleepiness can also be picked up. These indicators include hand movement, reaction time and heart-rate.

An overview of detecting sleepiness, or drowsiness, from sensor data is provided in the paper "Detecting Driver Drowsiness Based on Sensors: A Review", by Arun Sahayadhas, Kenneth Sundaraj and Murugappan Murugappan (included herein by reference).

For example, a drowsy person displays a number of characteristic facial movements, including rapid and constant blinking, nodding, or swinging their head, and frequent yawning. Computerized, non-intrusive, behavioral approaches may be used for determining the drowsiness level. For example, sleepiness unit 221 may determine, the so-called PERCLOS, which is the percentage of eyelid closure over the pupil overtime and reflects slow eyelid closures ("droops") rather than blinks. A sleepiness or drowsiness indicator may be obtained from a camera that registers the user face. In an embodiment, the camera comprises an Infra-Red Illuminator.

Sleep parameters of last night may be obtained by an external device, e.g., a sleep sensor, movement sensor, ECG sensor, etc. Sleep parameters include total time slept, amount of awakenings, sleep quality but also usual time awaken vs today awaken. For example, one can reasonably assume if a person today woke up at 6:00 while he normally wakes up at 8.00: he is sleepy in the first 30-60 minutes. For example, a sleepiness indicator may be increased if a user is interacting with communication device 200 a time period before a determined usual wake time. The time period may be user state dependent, or configurable.

A sleepiness indicator may be a number, e.g., a number on a scale from 0 to 10, or a number between 0 and 1, etc. For example, a higher number may indicate a higher level of sleepiness, or vice versa.

Indicator unit 220 may comprise a **mood unit 222.** User mood is also a useful user indicator that may be derived from sensor data 211. The selecting of the set of notification items may dependent upon the one or more user mood indicators. For example, an algorithm may take as input a notification item and a user state, e.g., the one or more user mood indicators, and compute an output that indicates if the notification item is selected. The output may be a weight that may be compared to a threshold weight.

User mood detection may use so-called automated emotion recognition (AEE). Mood may be detected from contactless sensors, e.g., cameras, and contact sensors, e.g., dry sensors.

For example, Facial Action Coding System (FACS) may be used to recognize emotions. For example, **mood unit 222** may be configured to track body posture, facial expressions, and/or gestures based on specific reference points, e.g., using a computer vision system. Using sensors, heart activity may be recorded. For example, ECG can analyze heart activity to recognize emotional factors. From heart data, one may also determine heart rate variability. Heart rate variability determines beat-to-beat changes in heart rate. The amplitude of a GSR signal can be related to emotions such as stress, anger, or excitement. In research, GSR measurements are often correlated to self-reported assessments of arousal. GSR is not under conscious control, and is driven by the sympathetic nervous system. Accordingly, skin conductance may be taken as a correlate to a degree of emotional arousal.

For example, in an embodiment, a front facing camera and dry electrodes are attached to the device to measure the heartrate and galvanic skin response (GSR) of the user to give an indication of the mood.

A machine-learning model may map sensor data to mood.

Note that the data used to determine sleepiness and to determine mood overlaps to a large extent. Accordingly, sleepiness detector 221 and mood detector 222 may in part use the same code and/or hardware. For example, both may use a computer vision system to track. Sleepiness detector 221 may use facial reference tracking data to identify sleepiness indicators, while mood detector 222 may use the facial reference tracking data to identify mood indicators.

References is made to the paper "Human Emotion Recognition: Review of Sensors and Methods" by Andrius Dzedzickis, Artu̅ras Kaklauskas, and Vytautas Bucinskas. The paper describes various ways to determine user mood from sensor data.

A particularly versatile machine learnable model for mapping sensor data to mood and/or sleepiness are neural networks. For example, in an embodiment, a long short-term memory may be provided with a stream of sensors data, possibly coming from different sensors, possibly as available. The long short-term memory may generate a current estimate of, e.g., sleepiness and/or mood, and/or other parameters of interest, heart rate, fatigue, health, and so on.

Communication device 200 may comprise a **notification items obtainer 230** configured to obtain a set of notification items for the user; shown is a set of notification items 231.

A notification may comprise an information item for display to the user. The user may take action based on the notification, e.g., now or later.

For example, a notification may inform the user about the technical state of communication device 200, which may prompt the user to make technical modifications to communication device 200. For example, a notification may include such information as: full storage, nearly full storage, virus detected, account not synchronizing, cloud not reachable, device offline, and so on. For example, upon reviewing a notification about full storage, the user may initiate to offload some items from communication device 200 to free storage; For example, upon reviewing a notification about a virus, a user may start a full scan for viruses, delete an infected item and so on. Action on a notification may be important for correct future functioning of the device, but may be ineffective if the operator of the device is not in a fit state to receive. As an extreme example, if the user is asleep say, then displaying a notification is fully ineffective. However, also if the user has a partial ability to attend to a notification than still it may be better to choose a more opportune time to deliver the information.

Notifications may be obtained through various mechanisms. For example, in an embodiment, communication device 200 comprises a notification interface configured for accessing notification items on the communication device. Obtaining the set of notification items may use the notification interface. For example, the notification interface may be an API, an operating system call, or the like. For example, an operating system may collect notification items, and offer an interface to access pending notification items. Notification items obtainer 230 may itself be part of an operating system. Notification items obtainer 230 may cooperate with a part outside of the operating system, e.g., a sensor system and/or a selector. Interestingly, all or most of the notification handling may be implemented in the operating system. Incorporating in the operating system, the determination of user state, collection of notification and their selection has the advantage of easier implementation, but on the other hand has the drawback the embodiment is limited to systems in which access may be obtained to the operating system. Alternative implementations without such access are discussed herein.

Notification items obtainer 230 may be configured to collect notification items from multiple sources. Notification items obtainer 230 may be part of, or external to, the operating system of communication device 200. For example, communication device 200 may comprise a communication interface configured for receiving communication data for the user, and/or a storage configured for storing user data associated with the user. For example, notification items obtainer 230 may be configured to derive from the received communication data and/or the stored user data the set of notification items. For example, when an incoming e-mail is received, a notification item may be created to reflect this.

A typical communication device 200, e.g., a smartphone, often has multiple sources of notifications. For example, the notification items may include one or more of an agenda notification, a notification sent by an app installed on the communication device, a received e-mail message, a received communication message, and so on.

In an embodiment, notification items obtainer 230 may be configured for agenda item detection, e.g., as used in conventional smartphones in which mails, meetings, messages, etc., are saved.

For example, in an embodiment, a notification item source, e.g., an app installed on communication device 200 may send a notification item to the operating system. The operating system may use an embodiment to select which notification item to show, or how to order the notification items, etc. In an embodiment, the operating system may show all notifications, but an embodiment masks some notification from viewing by the user. In an embodiment, the operating system may enable an embodiment to disable push notifications from specific apps, or other apps. This API may also be used to control the visibility of notification items.

Communication device 200 may comprise a **selector 240.** Selector 240 is configured for selecting from the set of notification items a notification item 241, the selecting being dependent upon indicators. The selected notification item 241 is displayed to the user, e.g., on a display 250, e.g., a touch-screen display.

Selector 240 may select more than one notification item for displaying; for example, Selector 240 may deselect one or more notification item to avoid displaying the deselected notification items. Selector 240 may even select all of the notification items, but change the order in which the notification items are to be displayed. In such embodiments, we will refer to the first displayed notification items as the selected notification item 241.

Selecting notification item(s) for display based on a user state 223 has the advantage that the notification items may be more effective, e.g., follow-up of a user upon receiving a notification may be poor if the user happens to be sleepy. Displaying the same notification items half an hour later when the user is fully awake may allow a more informed or adequate response from the user.

The selecting of the set of notification items may be dependent upon user state, e.g., one or more indicators, e.g., of sleepiness, mood, other physiological parameters, and so on. For example, an algorithm may take as input a notification item and a user state, e.g., the one or more user mood indicators, and compute an output that indicates if the notification item is selected. The output may be a weight that may be compared to a threshold weight.

In an embodiment, indicator dependent selection of notification items is only active during a time period, e.g., only from 22:00- 08:00. If a notification is received in this time period, the system may display it immediately if the user is up for it, but the notification may be delayed if this may give a better response. The time period may be configurable. Notifications with sufficient urgency may be able to override the system, and cause immediate display regardless of user state 223.

For example, if user state 223 indicates sleepiness, say, sleepiness scores an 8 out of 10, then a notification about a full storage may be delayed until sleepiness is back to normal levels. For example, if user state 223 indicates a mood qualified as negative, e.g., angry, depressed or the like, a notification of incoming e-mail may be delayed until mood is back to normal levels. Some embodiments offer high configurability, allowing a user to precisely set how mood and/or sleepiness and/or other indicators, should affect notification. Some embodiments may use default configurations, or notification profiles, etc.

There are many ways in which the selection of notification items can be implemented.

For example, in an embodiment, selector 240 uses a user state 223, e.g., a sleepiness indicator, and/or a mood indicator, to determine a maximal number of notification items from the set of notification items. For example, a lookup table may map user state 223 to a maximum number. Selector 240 may use the maximum number to select up to that many notification items. These may be the first notification items, until the maximum number is reached. These may also be ranked first before taken the first part thereof. The maximum number may increase with wakefulness, or mood neutrality. For example, if mood indicator(s) are away from average, the number of items shown may be lower.

For example, in an embodiment, selector 240 uses a user state 223, e.g., a sleepiness indicator, and/or a mood indicator, to prioritize notification items from the set of notification items. For example, notification items may be assigned a weight, and only notification items above a certain weight are selected and displayed. This ensures that limited cognitive capacity is used for the most important notification items. Not-selected notification items may be shown later.

For example, in an embodiment, selector 240 uses a user state 223, e.g., a sleepiness indicator, and/or a mood indicator, to order set of notification items 231. For example, with high wakefulness and/or average mood, the notification items may be shown in their natural order, e.g., as they are received by the operating system, or sorted by application, etc. However, if sleepiness and mood so indicate the order may be changed. For example, lighter, less important, or funny items, may be ordered to the front, so that notification items that need more attention are placed later in a notification queue.

For example, in an embodiment, communication device 200 and/or selector 240 is configured to assign a weight to notification items. In an embodiment, the weight of the notification items are compared to a threshold weight. For example, depending on user state 223, fewer high weight items may be shown, and/or low weight items may be shown first.

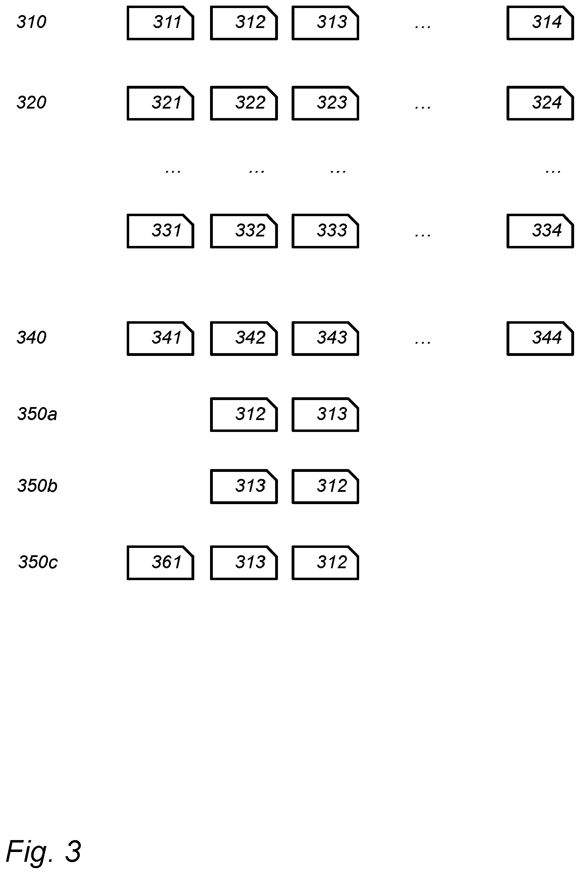

The weight of a notification item may be determined, at least in part, by applying a machine learned model to the notification item. An implementation of weight assignment is provided below with reference to Fig. 3.

For example, in an embodiment, when communication device 200 detects that the user starts using the device, communication device 200 may search for current agenda items that need the user's attention. At this point the user sleepiness detection and user mood detection may also be activated. These notification items may include but are not limited to emails, meeting requests, social media posts, and messages. For example, whenever these apps push a notification to the operating system, an embodiment may catch the notification item and adds the notification item to a pool for arrangement. These notification items are then rated for their impact on the user at that moment in time, where for instance work related items are weighed heavier than private items. Weighted items may need better user state before showing them. When the system has an indication on the mood and/or sleepiness of the user the system may select and/or arrange the notification items in an order that is deemed good for the user's early morning routine and stress level. For example, the system may show notification items gradually or in a sequence that is deemed convenient by the user.

**Fig. 3** schematically shows an example of an embodiment of a set of notification items 310. Shown are notification item 311-314. There may be more or fewer notification items than the four shown. For example, notifications items 311-314 may be received in that order, e.g., in a notification queue, e.g., in an operating system, or a notification handler, or the like.

For each notification items a set of notification features is determined. In Fig. 3, notification features, notification items, and weights are shown in the same column. For example, notification item 311 corresponds to notification features 321-331; notification item 312 corresponds to notification features 322-332; and so on. The set of notification features 320 may be determined by machine learned and/or handcrafted feature determiners. Examples of features include: sender, app, size, time of reception, word count, detected emotional content, number of pictures, and so on. Notifications features may be weakly correlated with a notification item's weight or a notification item's desirability for showing at a particular user state. By the same token notification features may be considerably easier to compute. However, by having multiple such features a machine learning model may tease out the signal from these features, to derive a weight. A notification item's weight may represent the expected impact a notification item may have on a user state, e.g., mood, sleepiness and the like. On the other hand, weight may also or instead represent an amount of cognitive attention, or time, a user is likely to expend on an item.

Based on the notification features a model assigns a weight. For example, the weight 341 is assigned to notification item 311 on the basis of features 321-331, etc. The model may well be a machine learnable model. Shown in Fig. 3 is the set of weights 340, with one weight for each notification item. Shown are weights 341-344, corresponding to notification items 311-314 respectively.

In rows 350a-350c, various selections of notification items are made depending on user state 223, e.g., based on a sleepiness indicator and/or mood indicator. For example, rows 350a-350c may be regarded as amended notification queue.

For example, in row 350a, a selection is made for two notification items: items 312 and 313. For example, these may be low-weighted notification items. For example, items 312 and 313 may be high-weighted items, but for this reason only 2 are selected, not all 4. In row 350b, the same notification items are selected, but row 350b indicates a new order for the notification items. For example, although item 312 may be received before item 313; nevertheless the system may show item 313 before item 312. For example, the system may determine to do so, if the weight of item 313 is lower than that of item 312. The missing notification items, e.g., 311, and 314 may be dismissed, but preferably are shown at a later time.

Row 350c illustrates a further possible feature that may be implemented in a communication device 200. A notification item 361 has been inserted that originally was not in the set of notification items 310. In this case, even after selection and reordering, the user state is deemed not sufficient to effectively handle items 312 and 313. Accordingly, a notification item 361 is obtained and inserted to cause the user state to be better adjusted to handling notification items; for example notification item 361 may be selected to reduce sleepiness, and/or to improve mood, e.g., return the mood to a mean mood value better suited to handle notification items. For example, suppose notifications items 313 refers to a full storage, and notification item 312 that the virus scanner seized scanning. These are important notification items, that the user ought to act upon. However, by bringing this notification items while the user is too sleepy, there is a non-negligible chance that a user may choose to ignore these, instead of interacting with the device to resolve these technical difficulties. Note that notification items do not necessarily relate to technical issues on communication device 200, they may also refer to technical issues at another device, e.g., a remote server for which the user is responsible.

For example, notifications that are considered more stressful items, e.g., as indicated by a weight such as weights 340, or as indicated by another indicator, e.g., an expected stress value, etc. For example, a work-related item, may be prepared for the user after lighter content. Notification items calculated to affect user state, e.g., sleepiness and/or mood, may be obtained from an external repository configured to store and providing notification items suitable for insertion in a notification stream as required.

For example, an embodiment may insert a good news notification item to a user on a day on which his/her user state indicates moodiness. Instead of going through notification items at the start, the user may be given some good news items for a good start of the day. After that, the system can gradually start giving the user the notification items, e.g., agenda items or the like.

Instead of the system checking the user's mood and sleepiness only in the morning, it could do this every time the user is using the device. If the system picks up that the user is sleepy or moody, it can show the user a funny short video, as can be found, e.g., on a social media platform. The system may then measure the user reaction to such a notification item, e.g., video, for example, with attached dry electrodes, and storing the measured user response. Overtime, the system can predict what will most elevate a user's mood and show a video finetuned on the user's preferences.

Determining a set of features for notification items, and training a machine learnable model on the features makes training easier and more predictable. For example, a machine learnable model such as SVM, decision trees, and the like, work well on a list of features. Determining features is not necessary. For example, a model may be trained directly on the notification items themselves, possibly together with the features. For such a training, a neural network is suited, amongst others.

Returning to Fig. 2. In an embodiment, communication device 200 may be configured to determine a user response to a displayed notification item. For example, user state 223 may be determined before selecting a notification item for display. However, user state 223 may also be determined after displaying the selected notification item 241 on display 250. In an embodiment, communication device 200 may comprise a **user response storage 260** configured to store displayed notifications items and the user response, e.g., the user state 223 after displaying the notification item. Further information may be recorded, e.g., information related to the displaying, for example, user state 223 prior to displaying the notification item; for example, features determined for the notification items (e.g., features show at 320 in Fig. 3).

Collecting user responses to notification items, and optionally, associated information, such as prior user state, or notification features like the notification source, etc. is useful as a machine learnable model may be trained or refined on them. For example, a machine learning unit may be comprised in communication device 200 that is configured to train a model on the training data in storage 260. The model may be previously trained on general data collected from other users, in which case the model may be fine-tuned for this user. The model may instead be trained from scratch. Training a model may use, e.g., simulated annealing, hill climbing, back propagation and similar training techniques. In an embodiment, machine learning unit is configured with the Adam optimization algorithm, e.g., a stochastic gradient descent optimization algorithm for machine learning.

The trained model may be used to predict user response to a notification before actually showing the notification item. This may be used during the selection of notification items. For example, a notification item with a predictable bad response may be delayed. Not showing a notification item does not mean the notification item is never showed, but by selecting a more propitious time the chances that the notification item is adequately acted upon is increased. For example, the system may learn that for a particular user, system maintenance notification predictably leads to a drop in mood, as a result of which the notification item is not acted upon. Showing the same notification item later in the day, may therefore lead to an improved interaction between the user and the device. For another user, device maintenance may not have the negative connotations, while, say, social network notifications do. For such a user, a different selection of notification items will thus result, which will lead to a better notification system for him/her.

User response may also be monitored to adjust the showing of notification items. For example, in an embodiment a user response to a displayed notification item may be determined, and, optionally stored in storage 260. Displaying the next notification item may be dependent upon said determined response. For example, if user state 223 becomes less favorable for handling notification items, the selection of notification items may be adjusted. For example, the number of selected notification items may be reduced. For example, a threshold weight for selected notification items may be adjusted so that only highly important, but fewer, notification items are shown, or conversely, so that only low-weight, less important notification items as shown.

The threshold weight and/or the assigned weight may depend on the origin of a notification item and the impact on the user's current physiological state that the notification item is likely to have.

Also in this area, a machine learning model may advantageously be applied. The current state 223 as well as the notification and/or its associated feature vector may be an input to a model predicting user state after the displaying.

In this respect, as particularly interesting application of an embodiment is to use a GSR sensor in the sensor system. Galvanic response is particularly interesting sensor as the galvanic response is not subject to conscious control, and is known to be correlated to emotional state. Unfortunately, the galvanic response may be caused by factors unrelated to communication device 200, or the notification items it displays. A machine learning model is able to learn the signal contained in the galvanic response and separate it from its inherent noise signal.

For example, in an embodiment, communication device 200 is configured to determine a user response to a displayed notification item, to determine a set of features of for each notification item, a machine learnable model being trained to predict the user response from the determined features. The measured user response including a galvanic skin response.

Indeed, a difficulty with GSR is the interpretation/contextual awareness: GSR often rises and or falls, but this can have a plurality of reasons. This can be solved by understanding where the user is looking at. A module for capturing user response to the given media input, has the advantage that the system can learn what is a good organization, e.g., order, and/or selection, of such notification items. This may use the same techniques described herein for the sleepiness detection and user mood detection units. Communication device 200 may capture the change in sleepiness or mood of the user during the handling of a notification item.

Communication device 200 may comprise a **display 250.** The display may be a touch sensitive display such as may be found on a smartphone. A notification items may be shown on the display. In an embodiment, a notification may comprise text and/or one or more images. A notification items could comprise a video clip, although this is unusual. Typically, a notification item takes up a small part of the screen. For example, a notification may take up at most 25%, or at most 10% of the screen, e.g., in square centimeters. A notification item may be displayed autonomously over an application that the user is currently using. A notification item may be removed by clicking, swiping the notification item, etc. A notification item may disappear after a time period, e.g., after 2 seconds, or more, or less. A notification item may allow a user to switch to an application in which the notification item can be managed. For example, a notification of a new e-mail may allow the user to directly switch to an e-mail program so that, say, the e-mail can be read, or answered, or an appointment scheduled, or the like. Different notification items may have different associated actions. For example, a notification concerning storage space may connect to a system utility of managing storage.

An embodiment for handling notification items may be installed in a communication device. For example, this may be done by installing software on the communication device.

For example, a system for handling notification items may comprise software arranged to interface with a sensor system, e.g., through a sensor interface, for obtaining sensor data from a user of the communication device. The software may be configured to determine from the sensor data one or more indicators for sleepiness of the user, obtaining a set of notification items for the user, selecting from the set of notification items a notification item, the selecting being dependent upon the indicators, and for causing and/or allowing the displaying of the selected notification item to the user.

A communication device may comprise an operating system configured for receiving notification items, e.g., from multiple notification sources, e.g., multiple apps, etc. The communication device may have installed thereon software that interacts with the operating system according to an embodiment. For example, a system for handling notification items may be installed on the communication device, wherein the system for handling notification items may cooperate with the operating system. There are various ways in which a notification handling according to an embodiment may interact with an operating system.

For example, a software application on the communication device may be configured to disable notifications of selected notification item sources, thus causing selected notification items to display by the operating system. For example, the notification system may determine based on the user state that notifications coming from e-mail are not likely to be handled well, by the user in his/her current state, whereas other types of notification items are usually handled better. Accordingly, the system may block Outlook in the first hour after waking, while the system does not block, say, notification from a language learning app. In an embodiment, notification selection is done on a per-notification basis, but as in this example, notification selection may also be done on groups of notifications, e.g., on notification classified, say, per app.

In an embodiment, a software application on the communication device is registered with the operating system as a notification handler. For example, the operating system may export an interface to allow software application to handle notification management, display, or the like. For example, an operating system may allow a program to register as a client of a particular type, in this case as a notification handler. Registering as a program may require authentication and may be done through an interface. After registration as a notification handler, the operating system may offload management of notification items to the registered notification handler. For example, notification items received in the operating system may be forwarded to the notification handler. For example, the notification handler, may use an operating system interface to display selected notification items.

In an embodiment, the operating system displays all notification items. However, a software application on the communication device may disable or mask not-selected notification items. This embodiment avoids cooperating from the operating system.

In an embodiment, a software application on the communication device disables display of all notification items by the operating system, and causes selected notification items to display instead of the operating system. For example, all notification may be disabled through a first setting. The notification system may then cause selected notification items to be displayed anyway, e.g., by raising an urgency flag.

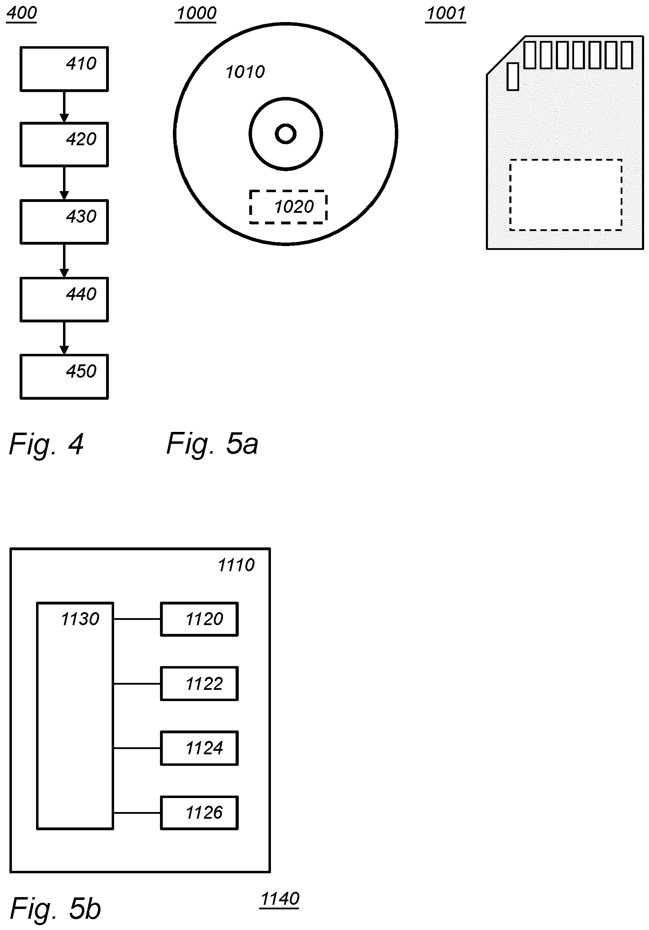

**Fig. 4** schematically shows an example of an embodiment of a method 400 for handling notification items. Method 400 for handling notification items may be executed on a communication device. Method 400 comprises
- obtaining (410) sensor data (211) through a sensor system from a user of the communication device,
- determining (420) from the sensor data one or more indicators for sleepiness of the user,
- obtaining (430) a set of notification items for the user,
- selecting (440) from the set of notification items a notification item, the selecting being dependent upon the indicators, and
- displaying (450) to the user the selected notification item.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 400. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

**Fig. 5a** shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform a method of handling notification items, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of handling notification items.

**Fig. 5b** shows in a schematic representation of a processor system 1140 according to an embodiment of communication device. The processor system comprises one or more integrated circuits 1110. The architecture of the one or more integrated circuits 1110 is schematically shown in Fig. 5b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the communication device, may comprise a processor circuit and a memory circuit, the processor being arranged to execute software stored in the memory circuit. For example, the processor circuit may be an Intel Core i7 processor, ARM Cortex-R8, Cortex-A78, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1140 is shown as including one of each described component, the various components may be duplicated in various embodiments. For example, processor 1120 may include multiple microprocessors that are configured to independently execute the methods described herein or are configured to perform steps or subroutines of the methods described herein such that the multiple processors cooperate to achieve the functionality described herein. Further, where the device 1140 is implemented in a cloud computing system, the various hardware components may belong to separate physical systems. For example, processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A communication device (110; 200) comprising
- a sensor system (210) configured for obtaining sensor data (211) from a user of the communication device,
- a processor system (130) configured for
- determining from the sensor data one or more indicators for sleepiness of the user,
- obtaining a set of notification items for the user,
- selecting from the set of notification items a notification item, the selecting being dependent upon the indicators, and
- displaying to the user the selected notification item.

2. A communication device as in Claim 1, wherein the processor system is configured for determining from the sensor data one or more physiological parameters for the user, the physiological parameters being indicators for sleepiness of the user.

3. A communication device as in any one of the preceding claims, wherein
- the communication device comprises a notification interface configured for accessing notification items on the communication device, the obtaining of the set of notification items being through the notification interface, and/or wherein
- the communication device comprises a communication interface configured for receiving communication data for the user, and/or a storage configured for storing user data associated with the user, the processor system being configured to derive from the received communication data and/or the stored user data the set of notification items.

4. A communication device as in any one of the preceding claims, wherein
- the selection is configured to select a maximal number of notification items from the set of notification items, wherein the number depends on the indicators, and/or
- the selection is configured to prioritize notification items from the set of notification items, wherein the prioritization depends on the indicators, and/or
- the selection is configured to order the set of notification items or the selected set of indicators, wherein the order depends on the indicators.

5. A communication device as in any one of the preceding claims, wherein the processor system is configured to assign a weight to notification items, selecting notification items comprises comparing the weight of the notification items to a threshold weight, wherein the threshold weight and/or the assigned weight depend on the indicators.

6. A communication device as in Claim 5, wherein the weight of a notification item is determined, at least in part, by applying a machine learned model to the notification item.

7. A communication device as in any one of the preceding claims wherein the notification items include one or more of agenda notification, notification sent by app installed on the communication device, e-mail messages, communication messages.

8. A communication device as in any one of the preceding claims, wherein the processor system is configured for indicator dependent selection of notification items only during a time period.

9. A communication device as in any one of the preceding claims, wherein
- the sensor system comprises a camera arranged to record an image or video of a face of the user, the processor system being configured to identify in the recording one or more indicators of sleepiness,
- the sensor system comprises a dry electrode sensor arranged to record one or more of heartrate, galvanic skin response (GSR), ECG,
- the sensor system comprises a sleep sensor and/or activity tracker configure to determine sleep quality in a night preceding the displaying of the notification item, and/or
- the sensor system comprises a photoplethysmography sensor, and/or
- the sensor system is configured to measure reaction time.

10. A communication device as in any one of the preceding claims, wherein one or more indicators of a user mood is derived from the sensor data, the selecting of the set of notification items being dependent upon the one or more user mood indicators.

11. A communication device as in any one of the preceding claims, wherein the sensor system and processor system are configured to determine a user response to a displayed notification item, wherein
- displaying a next notification item is dependent upon said determined response, and/or
- a machine learning algorithm is applied to multiple determined responses to obtain a trained model for predicting user response.

12. A communication device as in any one of the preceding claims, wherein the sensor system and processor system are configured to determine a user response to a displayed notification item, wherein the processor system is configured to
- determine a set of features of for each notification item, a machine learnable model being trained to predict the user response from the determined features.

13. A communication device as in any one of the preceding claims, wherein an indicator, an indicator of sleepiness, and/or an indicator of mood are derived from sensor data by applying a machine learnable model thereto.

14. A communication device as in any one of the preceding claims, wherein
- the sensor system comprises one or more sensors,
- the sensor system comprises a sensor interface configured to interface with one or more external sensors, e.g., a wearable sensor.

15. A communication device as in any one of the preceding claims comprising an operating system receiving the notification items, wherein
- a software application on the communication device is configured to disable notifications of selected notification item sources, thus causing selected notification items to display by the operating system, and/or
- a software application on the communication device is registered with the operating system as a notification handler, and/or
- the operating system displays all notification items, wherein a software application on the communication device disables or masks not-selected notification items, and/or
- a software application on the communication device disables display of all notification items by the operating system, and causes selected notification items to display instead of the operating system.

16. A method (400) for handling notification items on a communication device (110; 200), the method comprising
- obtaining (410) sensor data (211) through a sensor system from a user of the communication device,
- determining (420) from the sensor data one or more indicators for sleepiness of the user,
- obtaining (430) a set of notification items for the user,
- selecting (440) from the set of notification items a notification item, the selecting being dependent upon the indicators, and
- displaying (450) to the user the selected notification item.

17. A transitory or non-transitory computer readable medium (1000) comprising data (1020) representing instructions, which when executed by a processor system, cause the processor system to perform the method according to Claim 16.
